# EUROPEAN PATENT SPECIFICATION

(11) **EP 0 858 991 B1**
(45) Date of publication and mention of the grant of the patent: **16.01.2002**
(21) Application number: 98300911.9
(22) Date of filing: 09.02.1998
(51) Int. Cl.: C07C 68/04, C07C 68/00

(54) **Method for preparing diaryl carbonates employing hexaalkylguanidinium halides**
Verfahren zur Herstellung von Diarylcarbonate unter Verwendung von Hexaalkylguannidiniumhalogeniden
Procédé de préparation de carbonates de diaryle en utilisant des halogénures d'hexaalkylguanindinium

(30) Priority: 13.02.1997 US 40300 P; 27.08.1997 US 40264; 12.09.1997 US 929000
(43) Date of publication of application: 19.08.1998
(73) Proprietor: GENERAL ELECTRIC COMPANY, Schenectady, NY 12345 (US)
(72) Inventor: Pressman, Eric James, East Greenbush, New York 12061 (US); Shafer, Sheldon Jay, Clifton Park, New York 12065 (US)
(74) Representative: Szary, Anne Catherine, Dr.

(56) References cited:
- US-A- 5 231 210
- US-A- 5 284 964
- US-A- 5 399 734
- CHEMICAL ABSTRACTS, vol. 120, no. 1, 3 January 1994 Columbus, Ohio, US; abstract no. 8293q, P.GROS ET AL.: "selective esterification reaction involving hexaalkylguanidinium chloride catalysts" page 914; XP002060863 & SYNTH. COMMUN., vol. 23, no. 13, 1993, pages 1835-42,

## Description

This invention relates to the preparation of diaryl carbonates by carbonylation. More particularly, it relates to the improvement of diaryl carbonate yield in the carbonylation reaction.

Diaryl carbonates are valuable intermediates for the preparation of polycarbonates by transesterification with bisphenols in the melt. This method of polycarbonate preparation has environmental advantages over methods which employ phosgene, a toxic gas, as a reagent and environmentally detrimental chlorinated aliphatic hydrocarbons such as methylene chloride as solvents.

Various methods for the preparation of diaryl carbonates by a carbonylation reaction of hydroxyaromatic compounds with carbon monoxide and oxygen have been disclosed. In general, the carbonylation reaction requires a rather complex catalyst. Reference is made, for example, to US Patent 4,187,242, in which the catalyst is Group VIIIB metal, i.e., a metal selected from the group consisting of ruthenium, rhodium, palladium, osmium, iridium and platinum, or a complex thereof.

Further developments in the carbonylation reaction, including the use of such cocatalysts as cobalt pentadentate complexes and terpyridines, are disclosed in US Patents 5,231,210, 5,284,964 and 5,399,734. These patents also disclose the use of quaternary ammonium or phosphonium halides, as illustrated by tetra-n-butylammonium bromide, as part of the catalyst package.

The above-identified patents relate in general to carbonylation methods in which the Group VIIIB metal (most often palladium) component of the catalyst is present at a relatively high level, typically on the order of 500 ppm of palladium based on hydroxyaromatic compound. A further patent, US 5,498,789, discloses the use of a catalyst comprising a mixture of elemental or combined palladium, a lead compound and a quatemary ammonium or phosphonium halide. Palladium levels are disclosed as being roughly equivalent to those noted above or, in some instances, considerably lower, typically on the order of 50 ppm. Yields are expressed conventionally (i.e., in percentage) or in terms of "turnover number", which is the number of moles of diaryl carbonate formed per gram-atom of palladium present.

The commercial viability of the carbonylation reaction would be greatly increased if product yield therein could be improved. The relatively low yields disclosed in the working examples of the above-identified patents include some in the single digits. It is evident, therefore, that methods to improve the yield are needed.

### SUMMARY OF THE INVENTION

The present invention is based on the discovery that hexaalkylguanidinium halides are extremely active as halide sources for carbonylation. Their use in amounts essentially equivalent to the amounts of quatemary ammonium halides previously employed leads to a significant increase in product yield (i.e., percentage of hydroxyaromatic compound converted to reaction products) and/or turnover number, often without a decrease in selectivity (i.e., the proportion of diaryl carbonate obtained as a percentage of all of said reaction products).

The invention, therefore, is a method for preparing a diaryl carbonate which comprises contacting at least one hydroxyaromatic compound with oxygen and carbon monoxide in the presence of an amount effective for carbonylation of at least one catalytic material including an effective proportion of a source of bromide or chloride, said source being at least one hexaalkylguanidinium bromide or chloride.

### DETAILED DESCRIPTION; PREFERRED EMBODIMENTS

Any hydroxyaromatic compound may be employed in the present invention. Monohydroxyaromatic compounds, such as phenol, the cresols, the xylenols and p-cumylphenol, are generally preferred with phenol being most preferred. The invention may, however, also be employed with dihydroxyaromatic compounds such as resorcinol, hydroquinone and 2,2-bis(4-hydroxyphenyl)propane or "bisphenol A", whereupon the products are polycarbonates.

Other essential reagents in the method of this invention are oxygen and carbon monoxide, which react with the phenol to form the desired diaryl carbonate.

Any catalyst system effective in the carbonylation reaction may be employed. One essential catalyst constituent is one of the Group VIIIB metals, preferably palladium, or a compound thereof. Thus, palladium black or elemental palladium deposited on carbon are suitable, as well as palladium compounds such as halides, nitrates, carboxylates and complexes involving such compounds as carbon monoxide, amines, phosphines or olefins. Preferred in most instances are palladium(II) salts of organic acids, most often C₂₋₆ aliphatic carboxylic acids, and palladium(II) salts of β-diketones. Palladium(II) acetate and palladium(II) 2,4-pentanedionate are generally most preferred.

The catalyst package preferably also includes an inorganic cocatalyst, and/or an organic cocatalyst of the type disclosed in the aforementioned US Patent 5,284,964. It is preferred to employ both an inorganic and an organic cocatalyst.

One genus of inorganic cocatalysts includes compounds of the type disclosed in the aforementioned US Patent 5,231,210; namely, complexes of cobalt(II) salts with organic compounds capable of forming complexes, especially pentadentate complexes, therewith. Illustrative organic compounds of this type are nitrogen-heterocyclic compounds including pyridines, bipyridines, terpyridines, quinolines, isoquinolines and biquinolines; aliphatic polyamines such as ethylenediamine and tetraalkylethylenediamines; crown ethers; aromatic or aliphatic amine ethers such as cryptanes; and Schiff bases. The especially preferred inorganic cocatalyst is a cobalt(II) complex with bis[3-(salicylalamino)propyl]methylamine, said complex hereinafter being designated "CoSMDPT".

It is also within the scope of the invention to employ as an inorganic cocatalyst constituent a lead compound. Suitable lead compounds include the oxides, carboxylates (e.g., acetates, propionates, oxalates), alkoxides, nitrates and sulfates as well as lead-containing complexes.

Suitable organic cocatalysts which may be employed include various terpyridine, phenanthroline, quinoline and isoquinoline compounds including 2,2':6',2"-terpyridine, 4'-methylthio-2,2':6',2"-terpyridine and 2,2':6',2"-terpyridine N-oxide, 1,10-phenanthroline, 2,4,7,8-tetramethyl-1,10-phenanthroline, 4,7-diphenyl-1,10,phenanthroline and 3,4,7,8-tetramethyl-1,10-phenanthroline. The terpyridines and especially 2,2':6',2"-terpyridine are generally preferred.

The chloride or bromide, preferably bromide, source employed according to the present invention is a hexaalkylguanidinium chloride or bromide of the type disclosed in US Patent 5,229,482, the disclosure of which is incorporated by reference herein. Included are the α,ω-bis(pentaalkylguanidinium)alkane salts. Salts in which the alkyl groups contain 2-6 carbon atoms are preferred, with hexaethylguanidinium bromide being especially preferred.

The proportion of Group VIIIB metal source employed is typically in the range of 5-800 ppm by weight of metal, based on hydroxyaromatic compound. Thus, both "high concentration" systems in which the level of Group VIIIB metal is in the range of 100-800 ppm, and "low concentration" systems in which it is in the range of 5-100 ppm, are within the scope of the invention. The preferred Group VIIIB metal is palladium and is present in an amount of 5-100 ppm by weight based on hydroxy aromatic compound.

In the "high concentration" system, for each gram-atom of Group VIIIB metal there is usually employed 0.1-5.0 and especially 0.5-1.5 gram-atoms of cobalt, 0.1-3.0 and preferably 0.3-1.0 moles of organic cocatalyst and 2-150, preferably 5-50, more preferably 20-50 moles of hexaalkylguanidinium halide.

In the "low concentration system, lead is typically in the amount of 10⁻⁴ to 10⁻¹, preferably 10⁻⁴ to 10⁻², mole per mole of hydroxyaromatic compound. For each gram-atom of Group VIIIB metal there is usually employed 1000-5000, preferably 2500-3500, moles of hexaalkylguanidinium halide.

Gas is supplied to the reaction mixture in proportions of 2-50 mole percent oxygen, with the balance being carbon monoxide. The gases may be introduced separately or as a mixture, to a total pressure in the range of 1.01-25.33 mPa (10-250 atmospheres). Reaction temperatures in the range of 60-150°C are typical. Drying agents, typically molecular sieves, may be present in the reaction vessel. In order for the reaction to be as rapid as possible, it is preferred to maintain the reaction pressure in accordance with the aforementioned US Patent 5,399,734 until conversion of the hydroxyaromatic compound is complete.

The diaryl carbonates produced by the method of this invention may be isolated by conventional techniques. It is often preferred to form and thermally crack an adduct of the diaryl carbonate with the hydroxyaromatic compound, as described in US Patents 5,239,106 and 5,312,955.

The invention is illustrated by the following examples.

### EXAMPLE 1

A constant composition gas flow reactor system, as disclosed in the aforementioned US Patent 5,399,734, was charged with 61.94 g (658 mmol) of phenol, 990.1 mg (3.21 mmol) of hexaethylguanidinium bromide, 123.3 mg (0.300 mmol) of CoSMDPT, 23.6 mg (0.101 mmol) of 2,2':6',2"-terpyridine and 67.5 mg (0.293 mmol, 516 ppm) of palladium(II) acetate. Molecular sieves, 41 g, were placed in a perforated polytetrafluoroethylene basket mounted to the stir shaft of the reactor.

The reactor was sealed and heated to 110°C, with stirring, and a mixture of 9.5 mole percent oxygen and 91.5 mole percent carbon monoxide was introduced at a flow rate of 344 ml/min and a pressure of about 4.15 MPa (41 atmospheres). Gas flow was continued for 2 hours, after which a portion of the reaction mixture was removed and analyzed by high pressure liquid chromatography. The yield of diphenyl carbonate was found to be 42%, and the selectivity to diphenyl carbonate was 94%.

In a control reaction, run under identical conditions using an essentially equivalent amount of tetra-n-butylammonium bromide in place of the hexaalkylguanidinium bromide, the yield of diphenyl carbonate was 38% with a selectivity to diphenyl carbonate of 94%. Thus, the method of this invention afforded diphenyl carbonate in higher yield without a loss in selectivity.

### EXAMPLE 2

The procedure of Example 1 was repeated, substituting palladium(II) 2,4-pentanedioate for the palladium(II) acetate. The yield of diphenyl carbonate was 38%, and the selectivity was 85%. In the control, the yield was 35% and the selectivity 85%, This again shows the yield advantage provided by the method of the invention.

### EXAMPLE 3

A constant composition gas flow system similar to that of Example 1 was charged with 60.88 g (635 mmol) of phenol, 3.1994 g (10.39 mmol) of hexaethylguanidinium bromide, 53.1 mg (0.237 mmol) of lead(II) oxide and 0.9 mg (0.0029 mmol, 5 ppm) of palladium(II) 2,4-pentanedioate. The reactor was sealed, pressurized with 40.7 KPa (4150 kg/m²) of carbon monoxide and 20.4 KPa (2075 kg/m²) of air and heated at 100°C, and a mixture of carbon monoxide (300 ml/min) and air (50 ml/min) was introduced over 6 hours. A sample removed at the end of that time was analyzed and showed a diphenyl carbonate yield of 11.4% and a palladium turnover number of 11,534. A control using tetra-n-butylammonium bromide instead of hexaethylguanidinium bromide showed a yield of 10.0% and a palladium turnover number of 8,970.

## Claims

1. A method for preparing a diaryl carbonate which comprises contacting at least one hydroxyaromatic compound with oxygen and carbon monoxide in the presence of an amount effective for carbonylation of at least one catalytic material including an effective proportion of a source of bromide or chloride, said source being at least one hexaalkylguanidinium bromide or chloride.

2. A method according to claim 1 wherein the catalytic material comprises palladium or a compound thereof and an inorganic cocatalyst.

3. A method according to claim 1 orclaim 2 wherein the hydroxyaromatic compound is phenol.

4. A method according to claim 2 wherein the inorganic cocatalyst is a cobalt(II) salt with an organic compound capable of forming a pentadentate complex.

5. A method according to claim 2 wherein the inorganic cocatalyst is a lead compound.

6. A method according to claim 1 orclaim 2 further comprising an organic cocatalyst which is a terpyridine, phenanthroline, quinoline or isoquinoline compound.

7. A method according to claim 1 wherein the source is a hexaalkylguanidinium bromide.

8. A method according to any preceding claim wherein the proportions of oxygen and carbon monoxide are 2-50 mole percent oxygen, with the balance being carbon monoxide.

9. A method according to any preceding claim wherein the proportion of palladium is 5-100 ppm by weight based on hydroxyaromatic compound.

10. A method according to any preceding claim wherein 0.5-1.5 gram-atoms of cobalt, 0.3-1.0 mole of organic cocatalyst and 20-50 moles of hexaalkylguanidinium halide are employed per gram-atom of palladium.

## Patentansprüche

1. Verfahren zum Herstellen eines Diarylcarbonats, umfassend das in Berührung bringen mindestens einer aromatischen Hydroxylverbindung mit Sauerstoff und Kohlenmonoxid in Gegenwart einer für die Carbonylierung wirksamen Menge mindestens eines katalytischen Materials, das einen wirksamen Anteil einer Quelle von Bromid oder Chlorid einschließt, wobei diese Quelle mindestens ein Hexaalkylguadiniumbromid oder -chlorid ist.

2. Verfahren nach Anspruch 1, worin das katalytische Material Palladium oder eine Verbindung davon und einen anorganischen Cokatalysator umfasst.

3. Verfahren nach Anspruch 1 oder 2, worin die aromatische Hydroxylverbindung Phenol ist.

4. Verfahren nach Anspruch 2, worin der anorganische Cokatalysator ein Cobalt(II)salz mit einer organischen Verbindung ist, die zur Bildung eines fünfzähnigen Komplexes in der Lage ist.

5. Verfahren nach Anspruch 2, worin der anorganische Cokatalysator eine Bleiverbindung ist.

6. Verfahren nach Anspruch 1 oder 2, weiter umfassend einen organischen Cokatalysator, der eine Terpyridin-, Phenanthrolin-, Chinolin- oder Isochinolin-Verbindung ist.

7. Verfahren nach Anspruch 1, worin die Quelle ein Hexaalkylguanidiniumbromid ist.

8. Verfahren nach einem vorhergehenden Anspruch, worin die Anteile von Sauerstoff und Kohlenmonoxid 2-50 Mol-% Sauerstoff, Rest Kohlenmonoxid betragen.

9. Verfahren nach einem vorhergehenden Anspruch, worin der Anteil von Palladium 5-100 Gew.-ppm, bezogen auf die aromatische Hydroxylverbindung, ist.

10. Verfahren nach einem vorhergehenden Anspruch, worin 0,5-1,5 g-Atome Cobalt, 0,3-1,0 Mol organischer Cokatalysator und 20 bis 50 Mole Hexaalkylguadiniumhalogenid pro g-Atom Palladium eingesetzt werden.

## Revendications

1. Procédé pour préparer un carbonate de diaryle, qui comprend le fait de mettre au moins un composé aromatique hydroxylé en contact avec de l'oxygène et du monoxyde de carbone en présence d'une quantité efficace pour une carbonylation, d'au moins un matériau catalytique comprenant une proportion efficace d'une source de bromure ou de chlorure, ladite source étant au moins un bromure ou un chlorure d'hexa-alkylguanidinium.

2. Procédé selon la revendication 1, dans lequel le matériau catalytique comprend du palladium ou un composé de ce dernier et un co-catalyseur inorganique.

3. Procédé selon la revendication 1 ou 2, dans lequel le composé aromatique hydroxylé est le phénol.

4. Procédé selon la revendication 2, dans lequel le co-catalyseur inorganique est un sel de cobalt (II) associé à un composé organique capable de former un complexe pentadentate.

5. Procédé selon la revendication 2, dans lequel le co-catalyseur inorganique est un composé du plomb.

6. Procédé selon la revendication 1 ou 2, comprenant en outre un co-catalyseur organique qui est une terpyridine, une phénanthroline, une quinoline ou une isoquinoline.

7. Procédé selon la revendication 1, dans lequel la source est le bromure d'hexa-alkylguanidinium.

8. Procédé selon l'une quelconque des revendications précédentes, dans lequel les proportions d'oxygène et de monoxyde de carbone sont 2-50 % en moles d'oxygène, le restant étant du monoxyde de carbone.

9. Procédé selon l'une quelconque des revendications précédentes, dans lequel la proportion de palladium vaut 5 à 100 ppm en poids par rapport au composé aromatique hydroxylé.

10. Procédé selon l'une quelconque des revendications précédentes, dans lequel on utilise, par atome-gramme de palladium, 0,5 à 1,5 atomes-grammes de cobalt, 0,3 à 1,0 mole de co-catalyseur organique et 20 à 50 moles d'halogénure d'hexa-alkylguanidinium.
